(19) Europäisches Patentamt
European Patent Office
Office européen des brevets

(11) **EP 2 696 201 A1**

(12) **EUROPEAN PATENT APPLICATION**

(43) Date of publication:
**12.02.2014 Bulletin 2014/07**

(51) Int Cl.:
***G01N 33/543*** *(2006.01)*

(21) Application number: **12173383.6**

(22) Date of filing: **25.06.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

• **Kieninger, Jochen**
**79117 Freiburg (DE)**
• **Flamm, Hubert**
**79286 Glottertal (DE)**
• **Urban, Gerald. A**
**79102 Freiburg (DE)**

(74) Representative: **Keller, Günter et al**
**Lederer & Keller**
**Patentanwälte**
**Unsöldstrasse 2**
**80538 München (DE)**

(71) Applicant: **Albert-Ludwigs-Universität Freiburg**
**79085 Freiburg (DE)**

(72) Inventors:
• **Aravindalochanan, Kuppusamy**
**79110 Freiburg (DE)**

(54) **Sensors for selective electrochemical detection of reactive oxygen species and reactive nitrogen species in fluid media**

(57) The present invention relates to the field of electrochemical sensors, design and transduction techniques. In particular, the invention relates to a sensor for a selective electrochemical detection of an analyte compound in a fluid, the sensor comprising:
a) a first working electrode;
b) a second working electrode covered by a second membrane coating.

The second working electrode is in proximity to a catalyst and the second membrane coating is permeable for the analyte compound, whereby the catalyst is capable of catalysing the conversion of the analyte compound into a detection compound. The detection compound can be detected by the first working electrode and by the second working electrode. The analyte compound is selected from the group consisting of reactive oxygen species and reactive nitrogen species.

**EP 2 696 201 A1**

**Description**

The present invention relates to the field of electrochemical sensors, measurement of signals obtained therewith and transduction techniques. In particular, the invention relates to a sensor for selective electrochemical detection of reactive oxygen species and reactive nitrogen species, preferably for the detection of reactive nitrogen species, for instance, for the detection of nitroxyl. The electrochemical sensors of the present invention are especially suitable for measurements in fluid media, especially in biological and non-biological aqueous media.

**Background Art**

Reactive oxygen species (ROS) and reactive nitrogen species (RNS) are believed to be constantly generated *in vivo* in a tissue environment. A broad range of ROS and RNS have been demonstrated to be essential for a number of biological processes such as signal transduction, ageing, apoptosis and tissue development. However, the possibility to study the formation and biological role of a number of ROS and NOS *in vivo* has been significantly restricted due to lack of suitable analytical methods. For instance, because of the lack of sufficiently sensitive and selective methods for the detection of HNO, not even the formation of this species *in vivo* could be reliably verified.

Accordingly, a selective method for detection and quantification of ROS and RNS in biological tissues would be highly beneficial for the investigation of *in vivo* processes. It is highly desirable to monitor the formation of such species which commonly have a short lifetime. Furthermore, a selective method for detection and quantification of short-lived ROS and RNS, for instance of HNO, will allow to verify their generation *in vivo.*

Despite nitroxyl (HNO) displays a number of pharmacologically beneficial properties, this compound from the group of ROS/RNS remains poorly understood and inadequately studied.

In the last decades nitric oxide (NO) has gained a remarkable significance in mammalian cell biology due to its multiple mediative roles in physiology and patho-physiology. Further research has investigated the significance of other nitrogen oxides ($NO_x$) as well as of nitrite, nitrate, peroxynitrite, nitrosonium ions, nitrosothiols, nitroxyl, etc. For instance, biological studies of peroxynitrite ($OONO^-$) have revealed that this species can cause DNA damage, cellular function impairment, apoptosis, etc. if present in increased concentration. Similarly, nitrite ions ($NO_2^-$) have been demonstrated to act as a reservoir for NO, which, in turn, regulates a number of processes such as platelet function, leukocyte recruitment, mitochondrial respiration, vascular tone and cardiac function. However, the significance of nitroxyl (HNO) and of nitroxyl anion ($NO^-$) has not been properly understood due to the unusual properties and inherent low stability of these species. HNO has been reported to have a very low acidity, $pKa(HNO/NO^-) \approx 11.4$. Therefore, under physiological conditions (pH of 7.4) this species is expected to be predominantly present in the form of HNO rather than as NO- (V. Shafirovich et al., Proc. Nat. Acad. Sci. USA, 99(11):7340-7325, 2002).

One of the earliest known properties of HNO is its high reactivity towards thiols and thiol proteins. HNO inhibits the aldehyde dehydrogenase enzyme by modifying the active site called cysteine thiolate. Inhibition of the enzyme tends to reduce the alcohol intake. This property of HNO has been exploited for treating alcohol addiction. Apart from that, administering HNO producing compounds has also been reported to prevent toxicity after ischemic reperfusion. Furthermore, the ability of HNO to increase muscular contraction and selective venodilation, which has been recently discovered, makes HNO a suitable drug for heart failure treatments. Those applications highlight the importance of HNO in biological reactions.

Though the therapeutic significance of HNO has been gradually investigated by administering HNO producing drugs (e.g. Angeli's salt, ($Na_2N_2O_3$)), there has been no unequivocal demonstration of the endogenous production of HNO in mammalian systems. The lack of evidence for endogenous production of HNO *in vivo* results inter alia from the fact that there are currently no efficient, selective and sufficiently sensitive analytical methods for detection and quantification of HNO in biological tissues.

It is, therefore, an object of the present invention to provide an appropriate analytical method for detection and quantification of HNO as well as suitable sensors. Such sensors and their use will significantly facilitate further studies on the endogenous generation and physiological functions of HNO in biological systems.

Evidence of endogenous synthesis and putative roles of HNO have been long standing questions due to the unavailability of suitable analytical techniques. High reactivity, unusual chemical properties and expected low concentration of HNO *in vivo* pose a considerable challenge in detecting and measuring HNO.

Prior to the discovery of its biological relevance, HNO was predominantly known as an intermediate released from some inorganic compounds, such as sodium nitrosyl (NaNO), or Angeli's Salt ($Na_2N_2O_3$), or by some organic compounds, for instance as a by-product of the Nef reaction (conversion of nitro compounds into the corresponding carbonyl compounds). HNO rapidly dimerizes into hyponitrous acid which, in turn, subsequently decomposes into nitrous oxide ($N_2O$) as shown below (P. A. S. Smith et al., J. Am. Chem. Soc., 82(21):5731-5740, 1960):

$$HNO + HNO \rightarrow (HON=NOH) \rightarrow N_2O + H_2O \qquad (1)$$

Dimerization of HNO is a thermodynamically favourable second order reaction (reaction constant k = 8 x $10^6$ $M^{-1} \cdot s^{-1}$).

$N_2O$ is a stable compound which can be detected and quantified using a gas chromatography-mass spectrometer (GC-MS). Consequently, by detecting and quantifying $N_2O$ using GC-MS, one can detect HNO in a sample and determine its concentration. (J. M. Fukuto et al., Biochem. Pharmacol., 43(3):607-613, 1992).

However, this analytical procedure suffers from several significant drawbacks which prevent it from being used in the bio-sample analytics. Independently from HNO dimerization, $N_2O$ is also assumed to be present in biological environments. Accordingly, a selective detection and quantification of $N_2O$ resulting from HNO in the presence of $N_2O$ from other sources poses a considerable challenge. Moreover, because dimerization of HNO is a second order reaction, the formation of $N_2O$ at low concentrations of HNO, which are typical for biological systems, might be too slow to enable an efficient HNO detection. Therefore, a practical implementation of this method for the detection of HNO in biological systems is rather difficult.

Albeit the highly favourable reaction of HNO with thiols and thiol proteins *in vivo* and *in vitro* is well-known, the products of this reaction have been only recently characterized (B. Shen et al., Biochemistry, 44(42):14030-14044, 2005). HNO was shown to induce thiol conversion to four distinct species: sulfinamides ($RSONH_2$), mixed disulfides (RSSR'), intramolecular disulfides, and intramolecular sulfinamides (RSONHR'). These products are detectable by a mass spectrometry method, for instance by electrospray ionization. In particular, sulfinamides ($RSONH_2$) were shown to be useful for the detection of HNO. The presence of these products would therefore indicate the presence of HNO.

This detection method has a good selectivity. However, in practice an excessive amount of thiols is required to produce a detectable amount of sulfinamides. Therefore, detection of the desired product requires a special sample preparation which renders this method unpractical for the detection and, in particular, for the exact quantification of short-lived HNO in biological tissues.

Another method for detection and quantification of HNO is based on its ability to quickly reduce certain Mn(III) porphyrins to Mn(II) porphyrins by nitrosylation (M. A. Marti et al., J. Am. Chem. Soc., 127(12):4680-4684, 2005):

$$HNO + Mn^{III}TPPS \rightarrow Mn^{II}TPPS(NO) \qquad (2)$$

This reaction can be monitored by following the change of absorbances at 468 nm ($\lambda_{max}$ of $Mn^{III}TPPS$) and 424 nm ($\lambda_{max}$ of $Mn^{II}TPPS(NO)$). Importantly, these spectral changes were not observed in the presence of NO donors. Accordingly, this method allows a discrimination between HNO and NO and, consequently, a quantitative determination of the HNO concentration in the presence of NO. However, this method is not applicable in an aerobic environment due to the quick oxidation of Mn(II) porphyrins back to Mn(III) porphyrins. Since oxygen is usually present in biological systems this method cannot be used in many biologically relevant environments.

The above drawback has been overcome by using membranes which are impermeable to molecular oxygen (e.g. xerogels) and by enhancing HNO diffusion through incorporation of amidoamine-organosilicon hydrophilic nano-domains (C. J. Brinker et al., Academic Press, 1st edition, May 1990). This method is suitable for selective detection of HNO and is least affected by an environment containing molecular oxygen (K. P. Dobmeier et al., Anal. Chem., 80(4): 1247-1254, 2008). The drawback of this method is that the Mn(II) porphyrins formed after nitrosylation cannot be oxidized back to Mn(III) porphyrins unless there is a supply of molecular oxygen. Therefore, each sample must be calibrated and can be measured only once. This technique exhibits a poor measurement resolution due to multiple sample preparation and calibration. Moreover, the xerogel-containing sample must be hydrated for 20 minutes in order to achieve a proper HNO nitrosylation.

Thus, the existing methods for detection and quantification of HNO described above suffer from one or more major drawbacks like poor selectivity, low sensitivity and detection limit, cumbersome embodiment, etc. Moreover, almost all of these techniques did not show the ability to be applicable in bio-analytics. The progress in understanding endogenous HNO synthesis, physiological properties, drug development and treatment have been primarily hampered by lack of reliable analytical methods for HNO detection and/or quantification. A couple of decades ago a similar trend was observed in the field of NO biology. The mediative roles of NO had been unveiled by the sequential invention of various suitable analytical techniques. Therefore, almost all review papers related to chemistry and biology of HNO emphasize the need for a valid HNO detection and quantification techniques in order to elevate the understanding of its physiological and therapeutical relevance (J. M. Fukuto et al., Chem. Bio. Chem., 6(4):612-619, 2005; J. C. Irvine et al., Trends Pharmacol. Sciences, 29(12):601-608, 2008; N. Paolocci et al., Pharmacology & Therapeutics, 113(2):442-458, 2007).

The present invention provides sensors and their use in a method for a selective detection and a selective quantification of HNO as well as of other reactive nitrogen species and reactive oxygen species.

A sensor of the present invention can be used for a selective electrochemical detection of an analyte compound in a fluid. The sensor comprises at least

a) a first working electrode and
b) a second working electrode covered by a second membrane coating.

The second working electrode is in proximity to a catalyst and the second membrane coating is permeable for the analyte compound. The catalyst is capable of catalysing the conversion of the analyte compound into a detection compound. The catalyst is located in an inner area comprising the second working electrode whereby said area is separated by the second membrane coating from the outer area.

The detection compound can be detected by the first working electrode and by the second working electrode. The detection compound and the analyte compound are selected from the group consisting of reactive oxygen species and reactive nitrogen species. Preferably, the detection compound is a reactive nitrogen species. The first and the second working electrodes generate signals which can be detected, measured and compared with each other.

Said sensor is preferably used for a selective electrochemical detection and quantification of reactive oxygen species and reactive nitrogen species in a fluid.

The term "reactive oxygen species" (ROS) relates to highly reactive metabolites of molecular oxygen, which are generated in a tissue environment. ROS can be free radicals, ions or molecules. Examples of ROS include, but are not limited to, superoxide ion radical ($O_2^-$), hydroxyl radical ($OH\cdot$), peroxide ($ROO\cdot$), alkoxyl radicals ($RO$), hydrogen peroxide ($H_2O_2$), organic peroxide ($ROOR'$), ozone ($O_3$), singlet oxygen ($^1O_2$), etc. According to the present invention, ROS are preferably selected from the group consisting of superoxide anion, hydrogen peroxide and hydroxyl radical.

The term "reactive nitrogen species" (RNS) refers to highly reactive metabolites containing at least one nitrogen atom. These metabolites are usually generated in a tissue environment. RNS include but are not limited to nitric oxide (NO), peroxy nitride ($OONO^-$) nitrous acid ($HNO_2$), nitrite ion ($NO_2^-$), nitrosonium cation ($NO^+$), nitroxyl (HNO) and nitroxyl anion ($NO^-$). Preferably, the reactive nitrogen species according to the present invention is nitroxyl or nitroxyl anion. Particularly preferred, the reactive nitrogen species according to the present invention is nitroxyl. An overview on reactive nitrogen species and reactive oxygen species can be found in the article of E. Novo et al. (E. Novo et al., Fibrinogenesis and Tissue Repair, 1; 5, 2008).

According to the present invention, the analyte compound is in a fluid. The term "fluid" used in the present application refers to gases, liquids and aerosols, preferably to liquids. In a more preferred embodiment of the present invention, fluid is an aqueous solution containing the analyte compound. In a particularly preferred embodiment of the present invention, fluid is a cell lysate, a cell culture medium or a sample of a body fluid, such as plasma, serum or liquid. When the fluid is a liquid, the analyte compound may be present in the fluid in a dissolved form, in the form of droplets or in the form of a solid dispersed in the fluid. Preferably, the analyte compound is dissolved in the fluid. If the analyte compound is not dissolved in the liquid, this will influence the measured values.

In yet another embodiment of the invention, fluid is a gas containing the analyte compound. When the fluid is a gas, the analyte compound may be in the form of a gas, vapour or in the form of an aerosol dispersed in the fluid.

The term "detection compound" as used in the present application relates to a species which is formed from the analyte compound by the action of the catalyst. The detection compound can be detected by the first working electrode and by the second working electrode. The term "detection compound" refers to radicals, ions and molecules. For instance, when the analyte compound is nitroxyl, the detection compound can be nitric oxide (NO). Since the second working electrode is covered by the second membrane coating, whereby an area comprising the catalyst is defined, in said area the analyte is converted to the detection compound. This results in different signals obtained from the second working electrode compared with the first working electrode.

According to the present invention the detection compound and, preferably, also the analyte compound are electrochemically active species. The term "electrochemically active species" as used herein refers to free radicals, ions or molecules capable of undergoing a electrochemical redox-reaction on the surface of a working electrode. Therefore, electrochemically active species can be directly detected by the working electrode. Examples of electrochemically active species include, but are not limited to, $H_2O_2$, NO, $O_2$ etc. Molecular nitrogen $N_2$ is an example of an electrochemically inactive species.

In a preferred embodiment of the invention, the fluid contains the analyte compound and the detection compound.

In a particularly preferred embodiment, the first working electrode is covered by the first membrane coating, the second working electrode is covered by the second membrane coating, the analyte compound is nitroxyl and the detection compound is nitric oxide. In this embodiment, the catalyst, which may be e.g. superoxide dismutase, is preferably incorporated into the second membrane coating.

The materials of the first working electrode and of the second working electrode are not particularly limited, as long as the working electrodes are suitable for detecting the detection compound. In the preferred embodiment of the present invention, the materials of the first working electrode and of the second working electrode are identical. However, in another embodiment of the invention, the materials of the first working electrode and of the second working electrode are different.

Preferably, the material of the first working electrode and of the second working electrode is selected from the group of electrically conducting materials, such as carbon (graphite), indium tin oxide, iridium oxide, nickel, platinum, silver and gold and alloys thereof. The material of the working electrodes is at least 95 wt.-% pure, preferably at least 97 wt.-% pure, more preferred at least 99 wt.-% pure, even more preferred at least 99.5 wt.-% pure and particularly preferred at least 99.9 wt.-% pure.

Preferably, the materials of the first working electrode and of the second working electrode are suitable for detection of the detection compound. In a particularly preferred embodiment of the present invention the materials of the first working electrode and of the second working electrode are suitable for detection of nitric oxide.

In some embodiments of the present invention, the materials of the first working electrode and the second working electrode may be coated by an electrochemically active metalized polymer coating, by a polymeric porphyrin-type material or by a doped polymer. Accordingly, when the detection compound is nitric oxide, the first working electrode and the second working electrode are coated with an electrochemically active metalized polymer coating, by a polymeric porphyrin-type material or a doped polymer which are suitable for detection of nitric oxide. Preferably, both working electrodes are coated with a metalized polymer coating suitable for detection of nitric oxide.

Polymeric porphyrin-type materials include, but are not limited to, polymeric porphyrins and polyphthalocyanines. These materials can contain central metal ions in a complexed form, preferably transition metal ions, more preferred ions of a metal such as nickel, cobalt, or iron. Preferred porphyrin-type materials for the working electrodes are polymeric metalloporphyrins or metalized polyphthalocyanines, most preferably polymeric metalloporphyrins, which are organic p-type semiconductors and have a relatively high conductivity. The corresponding materials are known to a person skilled in the art and are, for instance, described in US 5,603,820. Preferably, polymeric porphyrins are obtained by a polymerization or a copolymerization from monomeric porphyrins *N,N'*-di(5-p-phenylene-10,15,20-tri(3-methoxy-4-hydroxyphenyl)porphyrin, 1,10-phenantroline-4,7-diamine, and 5-p-(pyrole-1-yl)phenylene-10,15,20-tri-(3-methoxy-4-hydroxyphenyl)porphyrin with Fe, Mn, Co and Ni as central metal ions in a complexed form. Even more preferred, monomeric porphyrins include tetrakis(3-methoxy-4-hydroxyphenyl)porphyrin (TMHPP) and meso-5'-0-p-phenylene-2', 3'-0-isopropylidene uridine-tri(n-methyl-4-pyridinium)porphyrin (PUP).

Polymeric porphyrin-type materials can interact with the detection compound and cause a change of its normal redox potential on the working electrode. When the detection compound is NO, the redox potential of NO is changed. The current or charge generated thereby is sufficiently high to be used as an analytical signal.

Doped polymers are electrically conducting polymers and are well known in the prior art. An overview on electrically conducting polymers can, for instance, be found in H. Naarmann, Ullmann's Encyclopedia of Industrial Chemistry, 1992, 5th Edition, A21, p. 429, "Polymers, electrically conductive".

A doped polymer can be a self-doped conducting polymer, for instance, a self-doped conductive polyaniline such as a conductive polyaniline incorporating sulfonic acid groups in the backbone. This polymer has an advantageous conductivity and pH resistance and is therefore a preferred material for the working electrodes. The term "self-doped" means that a doping moiety is covalently bonded to the polymer being doped.

Alternatively, the doped polymer may be an externally-doped polymer. The term "externally-doped" means that the polymer is exposed to an added substance which can change the polymer's electrical conductivity, for instance an acidic solution can provide a hydrogen ion to dope a polyaniline polymer and can concurrently provide a counter ion that is ionically, but not covalently bonded to the polymer molecule. Other examples of suitable externally-doped polymer include polyvinylmetallocenes (e.g. ferrocene), polyacetylene doped with different metal redox centers and polypyrraline doped with different redox centers such as, e.g. methyl viologen. Polymeric substituted glyoximes may also be employed.

The term "metalized polymer coating" relates to a polymer coating which, in turn, is coated with a thin layer of a metal, such as nickel, platinum, silver or gold. Preferably, the metalized polymer coating used in the present invention has a thin layer of platinum or gold.

In one preferred embodiment of the present invention, the material of the second working electrode is a metalized polymer coating suitable for detection of nitric oxide. More preferred, the material of the first and of the second working electrodes is a metalized polymer coating suitable for detection of nitric oxide.

According to the present invention, the second working electrode is covered with the second membrane coating, whereby the second membrane coating is permeable to the analyte compound. The present application employs terminology for membranes as recommended by IUPAC, specified in the publication by W. J. Korros et al. (W. J. Korros et al., Pure & Appl. Chem. 68(7):1479-1489, 1996).

In one embodiment of the present invention, the second working electrode is covered by the second membrane coating and the first working electrode is not covered by any membrane coating. However, if the first working electrode is not covered by any membrane coating, there is no diffusion barrier on the first working electrode. In this case, the concentration of the analyte compound reaching the first working electrode differs from the concentration of the analyte compound reaching the second working electrode. Therefore, an appropriate correction of the detected signals becomes necessary.

Moreover, for in vivo applications, it becomes necessary to avoid any direct contact between electrodes of the sensor and the tissues. Thin membranes are optimal for these applications.

It is, therefore, preferred that the first working electrode is covered by the first membrane coating. In an even more preferred embodiment, the first membrane coating and the second membrane coating consist of identical materials and have the same uniform thickness, whereby the first membrane coating comprises no catalyst incorporated therein. The first membrane coating is permeable to the detection compound and, preferably, also to the analyte compound.

Preferably, the second membrane coating and, optionally, the first membrane coating has a uniform thickness of from 0.1 $\mu$m to 1000 $\mu$m, preferably from 1 $\mu$m to 100 $\mu$m, particularly preferred from 10 $\mu$m to 50 $\mu$m. The uniform thickness of the membrane coatings may be measured by the method ASTM D6132-08.

The second membrane coating and, optionally, the first membrane coating may be also obtained by electropolymerization of monomers such 1,2-diaminobenzene, 1,3-diaminobenzene, 2,3-diaminonaphthalene, 1,5-diaminonaphthalene, 1,8-diaminonaphthalene, 5-amino-1-naphthol or resorcin. In these embodiments, the second membrane coating and, optionally, the first membrane coating may have a uniform thickness of 0.1 nm to 10 nm, preferably from 1 nm to 10 nm. For instance, polymers obtainable by electropolymerization of diaminobenzenes such as 1,3-diaminobenzene, satisfy the purpose of the membrane coating at a uniform thickness of less than 10 nm. A low thickness of the membrane as well as optimal concentration of the catalyst, e.g. of SOD, enhance the sensor's detection limit, reduces the response time and optimizes sensitivity and linearity of the response.

The material of the second membrane coating and, optionally, the first membrane coating is not particularly limited as long as sufficient permeability to the analyte compound is provided. Thus, in one preferred embodiment, the second membrane coating contains at least one semi-permeable organic polymer. Suitable semi-permeable organic polymers are known to a person skilled in the art and include, but are not limited to, poly-2-hydroxyethylmethacrylate (polyHEMA), aminoalkoxysilane xerogel containing trimethoxysilyl-terminated poly(amidoamine-organosilicon)dendrimers as well as polymers of 1,2-diaminobenzene, 1,3-diaminobenzene, 2,3-diaminonaphthalene, 1,5-diaminonaphthalene, 1,8-diaminonaphthalene, 5-amino-1-naphthol and resorcin. Preferably, the semi-permeable organic polymer of the second membrane coating contains at least one from the group of polymers of 1,2-diaminobenzene, 1,3-diaminobenzene, 2,3-diaminonaphthalene, 1,5-diaminonaphthalene, 1,8-diaminonaphthalene, 5-amino-1-naphthol or resorcin.

Another suitable material of the second membrane coating and, optionally, the first membrane coating is sulfonated tetrafluoroethylene based polymer (PTFE), which is inter alia commercially available under the trademark Nafion®. PTFE is particularly suitable as a material of the second membrane coating and, optionally, of the first membrane coating if the detection compound is nitric oxide. PTFE is preferred due to its inherent ability to reduce the influence of anions such as nitrite and nitrate ions during the measurement.

In one preferred embodiment, the semi-permeable organic polymers of the second membrane coating and, optionally, the first membrane coating are permeable to at least nitric oxide.

The membrane coatings employed in the present invention may be microporous or homogeneous. A microporous membrane consists of a solid matrix containing pores having a diameter from 1 nm to 20 $\mu$m. A homogeneous membrane is a dense film through with a mixture of compounds may be transported by a gradient of pressure, concentration, or electrical potential. Thus, a membrane comprising hydrogel may not be homogenous but still porous and a membrane comprising an electropolymer is homogenous and porous.

In another preferred embodiment of the present invention, the second membrane coating and, optionally, the first membrane coating contains at least one diffusion limiting inert membrane. The diffusion limiting inert membrane is capable of substantially limiting the diffusion rate of the detection compound. Preferably, the diffusion limiting inert membrane reduces the diffusion rate of the detection compound by not more than 90 %, preferably by not more than 95 %, even more preferred by not more than 98 %, measured at a temperature of 25°C, in comparison to the arrangement without any diffusion limiting membrane.

The diffusion limiting inert membrane is further capable of decreasing the diffusion rate of molecular oxygen dissolved in the fluid. Typically, at room temperature the ratio of effective diffusion coefficient of molecular oxygen in a membrane coating comprised of e.g. hydrogel ($D_{(O2)Hydrogel}$) to that in the fluid such as PBS ($D_{(O2)PBS}$) is 1 : 7 i.e. $D_{(O2)PBS} * 14\% = D_{(O2)Hydrogel}$.

The diffusion limiting inert membrane can be composed of multiple polymers or of one single polymer. Polymers which are suitable for use in the diffusion limiting inert membrane include but are not limited to poly-2-hydroxyethylmethacrylate (polyHEMA), aminoalkoxysilane xerogel containing trimethoxysilyl-terminated poly(amidoamine-organosilicon) dendrimers, polymers of 1,2-diaminobenzene, 1,3-diaminobenzene, 2,3-diaminonaphthalene, 1,5-diaminonaphthalene, 1,8-diaminonaphthalene, 5-amino-1-naphthol and resorcin. polyHEMA is the particularly preferred material for use in the diffusion limiting inert membrane.

In another embodiment of the present invention, the second membrane coating and, optionally, the first membrane coating comprises at least one diffusion limiting inert membrane as specified above and at least one oxygen impermeable membrane. The impermeability of this membrane for molecular oxygen is influenced by the process of coating. The diffusion limiting membrane and the oxygen impermeable membrane can be coated separately. However, it is further possible to prepare a mixture of the materials of both membranes and subsequently coat this mixture onto the surface of the second working electrode and, optionally, of the first working electrode.

In a further embodiment, the second membrane coating consists of one oxygen impermeable membrane and the catalyst, for instance an enzyme, incorporated therein. The first membrane coating consists of one oxygen impermeable membrane. Accordingly, the second membrane coating and the first membrane coating comprise no additional diffusion limiting membranes. This embodiment is particularly advantageous, because it serves several purposes:

- both membrane coatings are oxygen impermeable;
- a direct contact between the working electrodes and tissues is prevented;
- the second membrane coating comprises the catalyst, for instance an enzyme, incorporated therein;
- no additional diffusion limiting membrane (e.g. a hydrogel membrane) to carry the catalyst such as an enzyme is employed.

The term "oxygen impermeable membrane" relates to a membrane which is substantially impermeable for molecular oxygen in the triplet-state ($^3O_2$), whereby molecular oxygen may be in the form of a gas or dissolved in a liquid. Preferably, the oxygen transmission rate of the oxygen impermeable membrane is below 100, more preferably below 50 cm$^3$/m$^2$/24 h/atm. The oxygen permeability of the membranes should be determined according to the standard method ASTM D1434. In another embodiment of the present invention, the second membrane coating contains at least one oxygen impermeable membrane. In yet another embodiment, the second membrane coating contains no oxygen impermeable membrane.

In another embodiment of the present invention, the second membrane coating and, optionally, the first membrane coating contains at least one xerogel. The term "xerogel" used herein relates to an open network formed by the removal of all swelling agents from a gel.

In one embodiment of the present invention, the semi-permeable organic polymers in the second membrane coating and, optionally, in the first membrane coating prevent electrochemical fouling of the second working electrode and, optionally, of the first working electrode.

The second working electrode is located in close proximity to the catalyst, whereby the catalyst is capable of catalysing the conversion of the analyte compound into the detection compound. Therefore, the detection compound formed can reach the second working electrode. In one embodiment, the catalyst is incorporated into the second membrane coating. For instance, the catalyst may be dispersed in the material of the second membrane coating and/or be covalently linked to it. The catalyst may further be attached to the inner surface of the second membrane coating, which is in close proximity or in contact with the second working electrode.

In another embodiment, the catalyst may be located between the second membrane coating and the second working electrode, for instance in form of a solution. In yet another embodiment, the catalyst is located between the second membrane coating and the second working electrode, whereby the catalyst forms a solid layer on the second working electrode.

According to the present invention, there is no catalyst in close proximity to the first working electrode. Furthermore, the detection compound which is formed in close proximity to the second working electrode does not reach the first working electrode. If the first working electrode is covered by the first membrane coating, the first membrane coating does not comprise any catalyst capable of catalysing the conversion of the analyte compound into the detection compound.

In some embodiments of the present invention, the detection compound which is formed in close proximity to the second working electrode might partially diffuse away into the fluid. In order to prevent the detection compound from reaching the first working electrode and thereby affecting the performance of the sensor (so-called cross-talking) it is required that the first working electrode and the second working electrode are spatially separated from each other.

The preferred distance between the first working electrode and the second working electrode depends on factors such as the desired dimension of the sensor, properties of the fluid as well as permeability of the second membrane coating and, optionally, of the first membrane coating to the detection compound. Preferably, the distance between the first working electrode and the second working electrode is between 1 and 20 mm, more preferred between 1.5 and 10 mm and particularly preferred between 2 and 5 mm, for instance about 2.3 mm.

In some embodiments, in particular, for *in vivo* measurements, if the detection compound is known to be harmful to biological tissues, the second membrane coating might comprise an additional membrane preventing the diffusion of the detection compound which is being formed in close proximity to the second working electrode into the fluid. The second membrane coating may further contain a membrane which is capable of converting the detection compound into a compound which is non-toxic to the biological tissues.

Thus, for instance, if the analyte compound is HNO and the detection compound is NO, NO may diffuse away from the second working electrode into the fluid, thereby affecting the tissues. Accordingly, in this embodiment, the second membrane coating preferably contains a membrane which prevents the diffusion of NO into the fluid or converts NO into a compound which is non-toxic for the tissues.

According to the present invention, the catalyst may be any compound which is capable of catalysing the conversion of the analyte compound into the detection compound. Preferably the catalyst is an enzyme, such as superoxide dismutase, for instance, Cu-Zn superoxide dismutase. In this particular embodiment, the enzyme is preferably incorporated into the second membrane coating. The choice of the Cu-Zn superoxide dismutase (SOD) is not particularly limited. Cu-Zn SOD employed in the present invention may, for example, be derived from bovine erythrocytes, bovine liver, human erythrocytes, horse radish, canine erythrocytes or be recombinant. The corresponding enzymes are well known to a person skilled in the art and commercially available for instance from Sigma-Aldrich (Sigma-Aldrich Co LLC, St. Louis, USA). In particular, when the analyte compound is nitroxyl and the detection compound is nitric oxide, it is preferred that the second membrane coating contains SOD. It is preferred to use an enzyme which is stable against inactivation.

In yet another embodiment of the present invention, the second membrane coating contains no enzyme, in particular no SOD. In this embodiment, the catalyst may be an organic or an inorganic compound, for instance a copper compound, preferably a salt of $Cu^{2+}$. The corresponding copper compound may be covalently linked to the second membrane coating so that no substantial leakage of the catalyst from the second membrane coating into the fluid takes place. Alternatively, the copper compound can be dispersed in the second membrane coating. Alternatively, the copper compound may be covalently attached to the inner surface of the second membrane coating or be present between the second membrane coating and the second working electrode, for instance in form of an aqueous solution, for example as an aqueous $CuSO_4$ solution.

If the conversion of the analyte compound into the detection compound further requires the presence of an additional compound, such as a cofactor, this additional compound may also be present in close proximity to the catalyst. In some embodiments, this additional compound may also be added to the fluid or be dissolved in the solution located between the second membrane coating and the second working electrode.

In one embodiment of the present invention, the fluid containing the analyte compound further contains the detection compound. For instance, the fluid may contain nitroxyl and nitric oxide.

In a further embodiment of the present invention, an additional catalyst is employed. In this embodiment the additional catalyst is in proximity to the first working electrode and to the second working electrode. Preferably, the additional catalyst is incorporated into the first membrane coating and into the second membrane coating. According to the present invention, the additional catalyst may be any compound which is capable of catalyzing the conversion of an interfering analyte compound into an interfering detection compound.

The term "interfering detection compound" as used herein relates to a species which is formed from the interfering analyte compound by the action of the additional catalyst. The interfering detection compound can be detected by the first working electrode and by the second working electrode. Accordingly, the term "interfering analyte compound" relates to a species which interferes with the measurement of the detection compound. For instance, if the analyte compound is HNO and the detection compound is NO, the interfering analyte compound may be $O_2^-$ and the interfering detection compound may be $H_2O_2$.

The additional catalyst is capable of converting the interfering analyte compound into the interfering detection compound. However, the additional catalyst is not capable of converting the analyte compound into the detection compound. Therefore, the embodiment employing the additional catalyst allows a selective detection of the analyte compound in the presence of the interfering analyte compound. For example, if superoxide dismutase is used as the catalyst, HNO is converted to NO and $O_2^-$ is converted to $H_2O_2$ by this enzyme. The additional catalyst, which may be an enzyme, is only capable of converting $O_2^-$ to $H_2O_2$. The first working electrode and the second working electrode are capable of detecting both NO and $H_2O_2$. Because only $H_2O_2$ but not NO is detected on the first working electrode, the undesired influence of $H_2O_2$ can be effectively eliminated during the detection of NO. Accordingly, the selectivity of the sensor is increased.

In one embodiment of the present invention, the first working electrode and the second working electrode are attached to a substrate which is preferably flexible. The flexible substrate may be made of an organic polymer, for instance of an organic polymer selected from the group consisting of polyacrylate, polyimide, polyester, polycarbonate homo- and copolymers. Polyimide is a particularly suitable material for the substrate. Because polyimide substrate is flexible, mechanically resistant, chemically stable and resistant to water absorption, it can be advantageously used for medical applications.

In yet another embodiment of the present invention, the substrate is hard. In this embodiment, the material of the substrate may be selected from crystalline materials such as quartz or silicon. Alternatively, hard polymeric materials can be used for this purpose. The thickness of the substrate is preferably from 10 $\mu$m to 3 mm, more preferred from 100 $\mu$m to 1 mm, particularly preferred from 200 $\mu$m to 700 $\mu$m.

In a further embodiment of the present invention, the substrate further comprises at least one electrical isolation layer. In this particular embodiment, the substrate may be selected from organic polymers, from silicon oxide or from silicon nitride.

In a further embodiment of the present invention, the first working electrode and the second working electrode may be housed in isolating and supporting electrode bodies, such as capillaries, for instance glass capillaries. The corresponding arrangement is known in the prior art and has, for instance, been described by Zhang et al. (Zhang et al. Electrochem. Commun, 4:11-16, 2002) and electrodes having such arrangement are commercially available from World Precision Instruments (World Precision Instruments Inc., Florida, USA). In this embodiment, the working electrodes are preferably carbon fibers which may have a diameter between 5 $\mu$m and 20 $\mu$m, for instance 7 $\mu$m and a length of between 10 mm and 30 mm, for instance 15 mm.

The working electrodes may be housed in capillaries such as glass capillaries. The glass capillary in which one of the working electrodes is housed may be coated with the Ag/AgCl layer to serve as the reference electrode. The glass capillary in which the other working electrode is housed may be coated with a metal layer, for instance with a platinum layer and serve as the counter electrode. In this embodiment, the second membrane coating and, optionally, the first membrane coating comprise PTFE. In this embodiment, the tip diameter of the working electrodes may be between 50 nm and 500 nm, for example 100 nm. Accordingly, the corresponding sensor can be advantageously used for measurements in small samples of liquids and tissues as well as at the single cell level.

As a person skilled in the art will readily recognize, for the measurement of the electrical signals produced by the first working electrode and the second working electrode, a counter electrode and, optionally, a reference electrode is required. For instance, when an amperometric detection method is used, the current between the first working electrode and the counter electrode (the first signal) and the second working electrode and the counter electrode (the second signal) are measured. When a potentiometric detection method is used, the voltage between the first working electrode and the counter electrode (the first signal) and the second working electrode and the counter electrode (the second signal) are measured. The difference between the first signal and the second signal corresponds to the concentration of the analyte compound in the fluid.

The counter electrode and the reference electrode can be used as external electrodes or be built within the sensor of the present invention. Thus, in one preferred embodiment, the sensor of the present invention comprises the counter electrode. In another preferred embodiment of the invention, the sensor of the present invention comprises the counter electrode and the reference electrode.

Counter electrodes are well-known to a person skilled in the art and are commercially available. Preferable materials of the counter electrode are platinum, gold or carbon (graphite), whereby platinum is particularly preferred.

Reference electrodes are well-known to a person skilled in the art and are commercially available. Reference electrodes, which are suitable for use in the present invention can be selected from the group consisting of mercury-mercury(I)chloride (calomel), silver-silver chloride or thallium amalgam-thallium(I) chloride. Preferably, the reference electrode is a silver-silver chloride (Ag/AgCl) electrode. The reference electrode can be used for the adjustment of a constant or of a scanning potential applied during the measurement.

The sensor of the present invention can also be employed for detection of the analyte compound in a gas phase. In this embodiment, the sensor is encapsulated in a gas-impermeable cell. The gas-impermeable cell is equipped with a gas-permeable membrane, which is permeable to the analyte compound. Thus, the analyte compound can pass the gas-permeable membrane and its concentration can be subsequently detected.

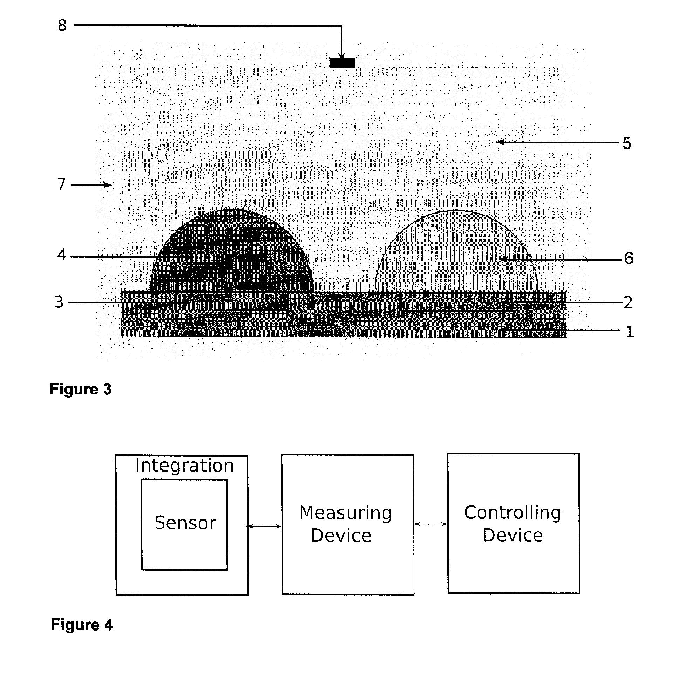

One preferred embodiment of a sensor for detecting the analyte compound in the gas phase is shown in Figure 3. In this embodiment, the sensor of the present invention comprises the following:

a) the substrate;
b) the first working electrode and the second working electrode, whereby the first working electrode is covered by the first membrane coating and the second working electrode is covered by the second membrane coating;
c) the second membrane coating comprises the catalyst incorporated therein, whereby the catalyst is optionally an enzyme;
d) the reference electrode for applying a constant or scanning potential;
e) the counter electrode for compensating the current measured by the working electrodes;
f) encapsulation with a gas permeable membrane to contain the electrolyte containing the analyte compound.

The first membrane coating as well as the second membrane coating may be electrically conductive. It is, however, preferred that the first membrane coating as well as the second membrane coating are not electrically conductive. Such membrane coatings may, for example, comprise hydrogel (polyHEMA).

In another embodiment, the sensor for detection of the analyte compound in a gas phase contains an additional catalyst. Thus, in this embodiment, the sensor of the present invention comprises the following:

a) the substrate;
b) the first working electrode and the second working electrode, whereby the first working electrode is covered by the first membrane coating and the second working electrode is covered by the second membrane coating;
c) the second membrane coating comprises the catalyst incorporated therein, whereby the catalyst is optionally an enzyme;

d) the first and the second membrane coatings comprise the additional catalyst incorporated therein, whereby the catalyst is optionally an enzyme;

e) the reference electrode for applying a constant or scanning potential;

f) the counter electrode for compensating the current measured by the working electrodes;

g) encapsulation with a gas permeable membrane to contain the electrolyte containing the analyte compound.

Also in this embodiment, the first membrane coating as well as the second membrane coating may be electrically conductive. It is, however, preferred that the first membrane coating as well as the second membrane coating are not electrically conductive. These membrane coatings may comprise hydrogel such as polyHEMA.

In one embodiment, the sensor for detection of the analyte compound in a gas phase contains no SOD coating on one of the working electrodes. In another embodiment, the sensor for detection of the analyte compound in a gas phase has no enzyme coating on one of the working electrodes. In yet another embodiment, the sensor for detection of the analyte compound in a gas phase has no counter electrode. In a further embodiment, the sensor for detection of the analyte compound in a gas phase has no encapsulation with gas permeable membrane. Yet, in a further embodiment of the present invention, the sensor for detection of the analyte compound in a gas phase has an encapsulation with gas permeable membrane to contain the electrolyte containing dissolved HNO.

Another aspect of the present invention relates to a method for producing a sensor for selective electrochemical detection of reactive oxygen species and reactive nitrogen species, such as HNO. Thus, this aspect of the invention relates to a method for manufacturing the sensor, the method comprising the following steps:

a) depositing at least one adhesion promoting layer on the substrate;

b) depositing at least two working electrodes on the adhesion promoting layers or on the substrate;

c) depositing at least one electrical isolation layer;

d) covering the first working electrode with a first membrane coating and the second working electrode with a second membrane coating, whereby the second membrane coating comprises a catalyst, preferably an enzyme.

Possible methods for depositing the adhesion promoting layer, materials of the first and second working electrode and of the electrical isolation layers are known in the prior art and are not particularly limited. In one of the preferred embodiments, the method of manufacturing the sensor comprises depositing of at least one adhesion promoting layer on the substrate by thin-film technology. In some embodiments, the thin-film technology is based on plasma deposition. Depositing materials of the first and second working electrodes on the adhesion promoting layer or on the substrate may be carried out by plasma based vacuum evaporation. Alternatively, depositing the materials of the first and second working electrodes may be carried out by galvanisation. In a further embodiment of the present invention, screen printing electrodes and laminating isolation layers are employed.

As a person skilled in the art will readily recognise, the methods for depositing the electrical isolation layer employed in the present invention are not particularly limited. For example, depositing electrical isolation layers may be carried out by plasma based vacuum evaporation.

Depositing the first and, optionally, the second membrane coatings may be carried out by a number of methods known in the prior art, for instance, it can be performed by electropolymerisation. Alternatively, depositing the first and, optionally, the second membrane coatings may be carried out by dispensing. In yet another embodiment, depositing the first and, optionally, the second membrane coatings is carried out by dispensing and a subsequent UV curing. Finally, in another embodiment, depositing of the first and, optionally, second membrane coatings is carried out by dispensing, followed by UV curing in oxygen reduced environment.

Another aspect of the present invention relates to the use of the sensor for selective electrochemical detection of the analyte compound in a fluid. The method of the present invention comprises measuring of the electrical signal from the first working electrode (the first signal) and the electrical signal from the second working electrode (the second signal) by any electrochemical detection technique, preferably, by an analytical method selected from the group consisting of amperometry, potentiometry, square wave voltammetry, differential pulse amperometry and differential pulse voltammetry.

In one embodiment, the corresponding method of the present invention comprises the steps of:

a) providing the sensor of the present invention to the fluid containing the analyte compound;

b) measuring the electrical signal produced between the first working electrode and the counter electrode (the first signal) and the electrical signal produced between the second working electrode and the counter electrode (the second signal), optionally upon using a reference electrode such as an Ag/AgCl electrode;

c) the difference between the first signal and the second signal corresponds to the concentration of the analyte compound.

Thus, when the concentration of HNO is to be determined, the method the present invention comprises the following steps:

a) providing the sensor to the fluid containing dissolved HNO;

b) measuring the electrical signals produced between the first working electrode and the counter electrode (the first signal) and the electrical signal produced between the second working electrode and the counter electrode (the second signal), optionally upon using a reference electrode such as an Ag/AgCl electrode;

c) the difference between the first signal and the second signal corresponds to the concentration of HNO in the fluid.

In general, the measurement can be attained by a three-electrode method employing the first working electrode, the second working electrode, and the reference electrode or the counter electrode. According to the amperometric measurement principle, a defined potential is applied between the working electrode and the counter or the reference electrode.

Alternatively, the measurement can be carried out by a four-electrode method employing the first working electrode, the second working electrode, the counter electrode and the reference electrode. According to the amperometric principle, here, a defined potential is applied between the working electrodes and the reference electrode. The counter electrode is used to balance the faradaic reaction occurring at the working electrode. The sole purpose of the counter electrode here is to stabilize the potential applied between the working and the reference electrode.

In some embodiments of the present invention, the fluid contains no dissolved HNO.

If an interfering analyte compound is present in the fluid, the undesired influence of the interfering detection compound may be eliminated by the choice of electrochemical measurement technique (e.g. square wave voltammetry). For example, during the measurement of HNO, the influence of $H_2O_2$ can be eliminated by using an appropriate electrochemical measurement technique e.g. square wave voltammetry. Alternatively, the undesired influence of the interfering detection compound may be eliminated by using an additional catalyst located in the first and in the second membrane coatings.

In summary, in the preferred embodiment, the present invention relates to a method of selective electrochemical detection and quantification of HNO. In the particularly preferred embodiment, the method of the present invention utilises an electrochemical measurement technique by a pair of identical working electrodes with nitroxyl distinguishing membrane coatings. The signal difference between these working electrodes is exclusively dependent on the presence of HNO. The principle described herein provides the advantage of real-time HNO measurement with high selectivity and sensitivity. Moreover, miniaturised sensor design provides an easy integration to measure HNO in tissue environment (e.g. *in vitro* and *in vivo*). Depending on the sensor embodiment, this principle is applicable for aerobic,. anaerobic, liquid and gaseous environment.

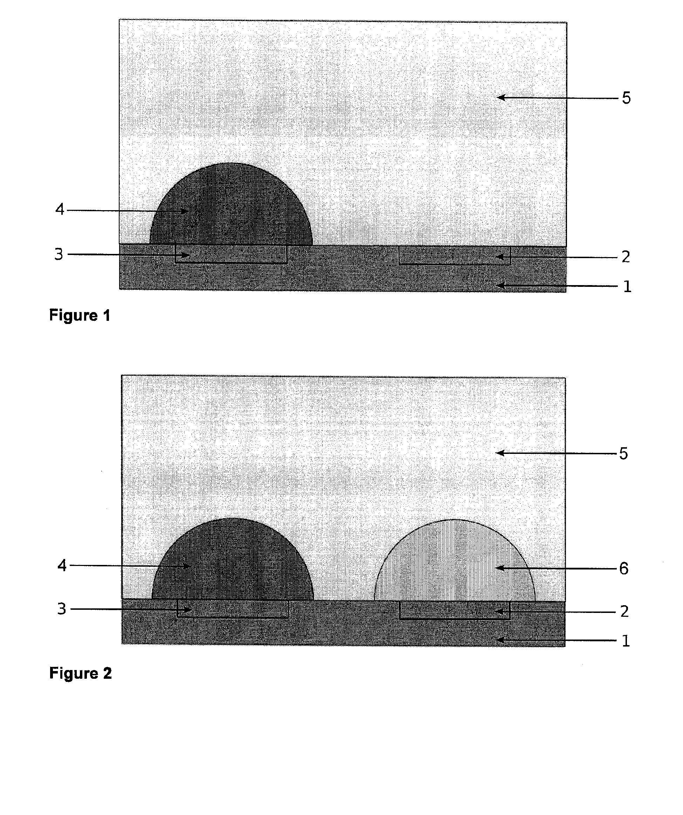

The invention will now be explained in detail on basis of Figures 1 to 8.

Figures 1 to 3 illustrate schematically preferred sensor of the present invention.

Figure 1 represents the general cross-sectional view of the sensor for a selective electrochemical detection of an analyte compound in a fluid.

1. Holding component (substrate and isolation layer)
2. First working electrode
3. Second working electrode
4. Second membrane coating
5. Fluid containing the analyte compound.

The sensor essentially consists of the holding component 1, the first working electrode 2 and the second working electrode 3 covered by the second membrane coating 4. The sensor is in contact with the fluid 5, containing the analyte compound. The holding component 1 acts as the substrate for the sensor, in particular for the first working electrode 2, the second working electrode 3 and the second membrane coating 4 and also electrically isolates the working electrodes 2 and 3. The substrate 1 can be either hard or flexible depending on the choice of application.

Preferably, the second membrane coating 4 comprises a catalyst, for instance an enzyme, which is capable of converting the analyte compound into the detection compound.

In general, the working electrodes 2 and 3 are miniaturized and are able to accomplish electrochemical detection and measurement of the detection compound, for instance of HNO or $NO_x$. Some examples of suitable materials of the working electrodes 2 and 3 include platinum, platinum-black, gold, glassy carbon electrodes, etc.

The sensor of the present invention can further comprise a counter electrode and/or a reference electrode (not shown). They can be built within the sensor or can be external electrodes for the measurement. The common choices of the counter and the reference electrodes are platinum and Ag/AgCl electrodes, respectively.

The sensor shown in Figure 1 can be used for detecting and quantifying HNO in anaerobic liquid media and for detecting HNO in aerobic liquid media.

Figure 2 represents the cross-section of view of a sensor for a selective electrochemical detection of an analyte compound.

1. Holding component (substrate and isolation layer)
2. First working electrode
3. Second working electrode
4. Second membrane coating
5. Fluid containing the analyte compound
6. First membrane coating.

The holding component 1 acts as the substrate for the sensor as well as for the first membrane coating 6 and second membrane coating 4. The first working electrode 2 is covered by the first membrane coating 6. The second working electrode 3 is covered by the second membrane coating 4. The first membrane coating 6 and the second membrane coating 4 can consist of a single or multiple membranes. The essential functions of the first membrane coating 6 are diffusion limitation, prevention of direct contact with tissues and cells and, if necessary, maintenance of low molecular oxygen concentration near the first working electrode.

Xerogels are suitable polymeric materials which are impermeable to molecular oxygen when coated on the electrode (C. J. Brinker et al., Academic Press, 1st edition, May 1990).

Therefore, xerogels can be used both for the first membrane coating 6 and for the second membrane coating 4.

When the selective electrochemical detection of the analyte compound is carried out in anaerobic liquid media, the permeability of the first membrane coating 6 and the second membrane coating 4 to molecular oxygen is not particularly relevant. However, when the sensor of the present invention is employed in aerobic liquid media, the permeability of the first membrane coating 6 and the second membrane coating 4 to molecular oxygen is preferably kept sufficiently low. Thus, the first membrane coating 6 and the second membrane coating 4 preferably contain oxygen impermeable membranes which may be present as separate layers. Alternatively, the first membrane coating 6 and the second membrane coating 4 may be prepared by coating a mixture containing one or several oxygen-impermeable components such as xerogels.

The embodiment shown in Figure 2 can be used for detecting HNO in aerobic and for absolute quantification of HNO in anaerobic liquid media. Preferably, the second membrane coating 4 contains a Cu-Zn SOD as catalyst.

Alternatively, this embodiment may be used for absolute quantification of HNO in aerobic liquid media when the first membrane coating 6 and the second membrane coating 4 are substantially impermeable to molecular oxygen.

Using oxygen impermeable membranes is particularly preferred when the sensor of the present invention is used for absolute quantification of HNO in aerobic environment. In this case, the analyte compound is HNO, the detection compound is NO and the catalyst is preferably a Cu-Zn superoxide dismutase. Because NO is rapidly oxidized by molecular oxygen in an oxygen-rich environment, the absolute concentration of HNO cannot be measured, when molecular oxygen is present in proximity to the working electrodes 2 and 3. Accordingly, oxygen impermeable membranes are incorporated into the membrane coatings 4 and 6 and therefore the concentration of molecular oxygen in proximity to the working electrodes 2 and 3 can be significantly reduced. Thus, the concentration of NO in proximity to the working electrodes 2 and 3 is no longer affected by molecular oxygen. In particular, the presence of xerogels enables a precise measurement of absolute NO concentration as an indicator for short-lived HNO in aerobic liquid media.

The second working electrode 3 is covered by the second membrane coating 4 which comprises in a preferred embodiment a definite concentration of Cu-Zn superoxide dismutase (SOD) enzyme. SOD enzyme is added due to its ability to convert HNO to NO (equation 3)

$$Cu(II)SOD + HNO \rightarrow Cu(I)SOD + NO + H^+ \qquad (3)$$

Among the pair of working electrodes 2 and 3 only the SOD coated electrode (the second working electrode 3) can read the NO signal, which has been converted due to the presence of HNO. Thus, the signals of the two working electrodes 2 and 3 differ when HNO is present. The difference in these signals is employed for detection or quantifying the concentration of HNO present in the fluid 5.

Figure 3 shows a cross-sectional view of the sensor for selective electrochemical detection of an analyte compound in gaseous media.

1. Holding component (substrate and isolation layer)
2. First working electrode
3. Second working electrode
4. Second membrane coating

5. Electrolyte containing the analyte compound
6. First membrane coating
7. Gas-impermeable cell
8. Gas-permeable membrane.

The first working electrode 2 is covered by the first membrane coating 6 and the second coating electrode 3 is covered by the second membrane coating 4. The sensor is encapsulated into a gas impermeable cell 7 equipped with a gas permeable membrane 8. Preferably, the first membrane coating 6 and the second membrane coating 4 are substantially impermeable to molecular oxygen. Preferably, the second membrane coating 4 contains a catalyst dispersed therein. In one of preferred embodiments, this sensor is used for absolute quantification of HNO in gaseous media. In this embodiment, the catalyst is an Cu-Zn SOD.

The sensor shown in Figure 3 may be used for the detection of HNO.

Figure 4 represents the principle of the total HNO detection and quantification system. It comprises a sensor, integration, measuring and controlling devices for a real-time measurement. The general embodiment of the sensor is as shown in Figures 1 to 3. Integration is the electrical connectivity between the sensor and the measuring device. The sensor can be custom integrated as per the application needed and it can be either single or multiple sensor integration. As outlined above, the sensor may further contain the counter electrode and/or the reference electrode (not shown) which are preferably built-in.

Some integration examples are presented in literature (see e.g. K. Aravindalochanan et al., Solid-State Sensors, Actuators and Microsystems Conference, 2007, Transducers 2007. International, pages 1907-1910; P. Ebbesen, Journal of Enzyme Inhibition and Medicinal Chemistry, 24 Suppl 1:1-39, 2009). The integration is further connected to the measuring device which acts as a potentionstat. The potentiostat is an analytical device which is able to control the working electrodes, the reference electrode and the counter electrode. The measuring device is further connected to the controlling device (e.g. computer) for data monitoring and storage.

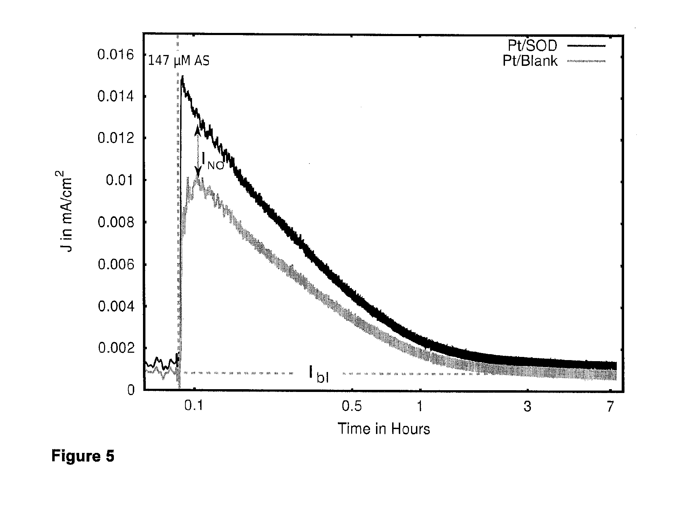

The result presented in Figure 5 illustrates the principle of the present invention and demonstrates the function of the HNO detection system. The grey and black curves denote the signal of the first working electrode coated with polyHEMA (Pt/Blank) and the signal of the second working electrode, which was coated with SOD enzyme mixed with polyHEMA (Pt/SOD), respectively. The occurring difference between the gray and black curves $I_{NO}$ is due to the catalytic conversion of HNO to NO by SOD enzyme at the Pt/SOD electrode.

Figure 6 illustrates the measurement of HNO formed by decomposition of Angeli's salt at different concentrations.

Figure 7 illustrates the selective measurement of HNO in the presence of nitrite ions $NO_2^-$. In particular, Figure 7 compares the sensor performance in PBS while adding the solution containing Angeli's salt (upper series) to the sensor performance in PBS while adding the solution containing sodium nitrite salt (lower series).

**Multiparameter monitoring**

As explained above, the sensor of the present invention allows detection and quantification of reactive nitrogen species (RNS) and reactive oxygen species (ROS), in particular of RNS such as nitrogen oxides ($NO_x$) and HNO. Each of these species is unique in its physiological and/or patho-physiological behaviour. However, if several ROS and RNS are present in one sample, a simultaneous measurement of their concentrations becomes a challenging issue. Accordingly, another aspect of the present invention provides a method for simultaneous measurement of concentrations of various ROS and RNS in a fluid.

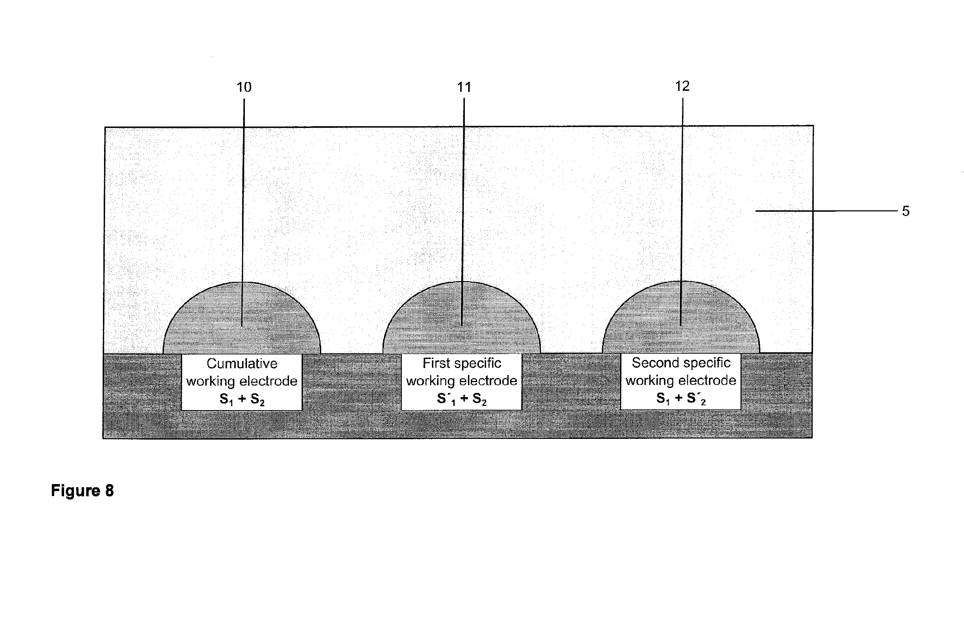

The principle of this aspect of the present invention is illustrated in Figure 8. The arrangement shown therein comprises three working electrodes and allows a simultaneous determination of concentrations of two species $S_1$ and $S_2$ in the fluid 5. Preferably, all three working electrodes are located within a single sensor. The cumulative working electrode is covered by the membrane coating 10. The membrane coating 10 comprises no catalyst. Accordingly, the cumulative working electrode delivers a signal corresponding to the total concentration of species $S_1$ and $S_2$ in the fluid 5.

The first specific working electrode is covered by the membrane coating 11. The membrane coating 11 comprises a catalyst which is capable of converting the species $S_1$ to the species $S'_1$. Preferably, the catalyst in the membrane coating 11 is not capable of converting the species $S_2$, Therefore, the first specific working electrode delivers a signal which corresponds to concentrations of species $S'_1$ and $S_2$.

The second specific working electrode is covered by the membrane coating 12. The membrane coating 12 comprises a catalyst which is different from the catalyst in the membrane coating 11. The catalyst in the membrane coating 12 is capable of converting the species $S_2$ into the species $S'_2$ and preferably is not capable of converting the species $S_1$. Therefore, the second specific working electrode delivers a signal which corresponds to concentrations of species $S_1$ and $S'_2$,

The difference between the signal of the cumulative working electrode and the first specific working electrode thus exclusively depends on the concentration of the species $S_1$. Similarly, the difference between the signal of the cumulative working electrode and the second specific working electrode only depends on the concentration of the species $S_2$.

In the following example, the species $S_1$ is nitrite ion ($NO_2^-$) and the species $S_2$ is nitroxyl (HNO). The measurements are carried out by amperometric detection method and thus the current between the working electrodes (the cumulative working electrode and the specific working electrodes) and the counter electrode (not shown) is measured. The catalyst in the membrane coating 11 may be an enzyme such as carbonic anhydrase. Carbonic anhydrase is an enzyme capable of converting nitrite ion to NO. NO is detected by the first specific working electrode and this results in the current due to the conversion of nitrite ion to NO ($S'_1 + S_2$). The current difference between the cumulative working electrode and the first specific working electrode thus exclusively depends on the concentration of nitrite ions in the fluid. Similar case can also be observed for HNO where the second specific working electrode is covered with the membrane coating 12 comprising an enzyme such as SOD. As already explained above, SOD is capable of converting HNO to NO. At any time, the differences between the signals from the first specific working electrode, the second specific working electrode and the cumulative working electrode allow to determine the concentration of nitrite ions and HNO, respectively.

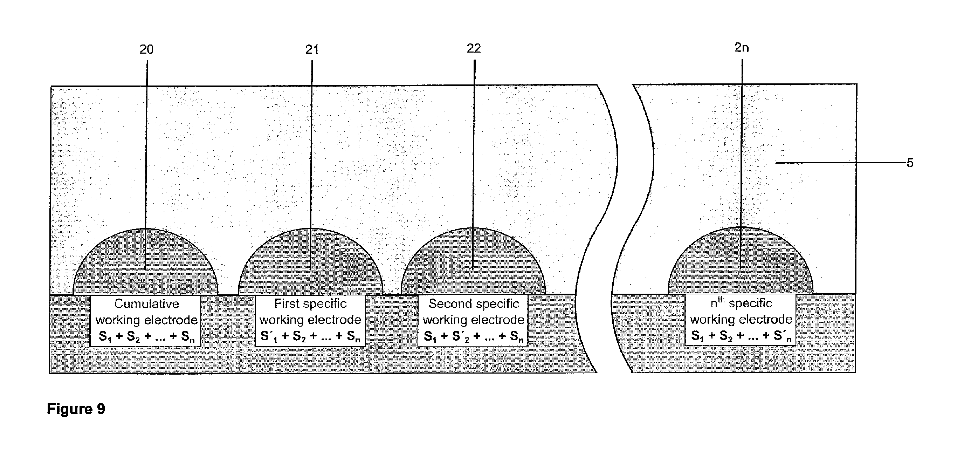

A more general embodiment of the present invention employing n+1 working electrodes is shown in Figure 9. Preferably, all n+1 working electrodes are located within a single sensor. The cumulative working electrode is covered by the membrane coating 20, which comprises no catalyst. The first specific working electrode is covered by the membrane coating 21, which comprises a catalyst capable of converting the species $S_1$ into the species $S'_1$. The second specific working electrode is covered by the membrane coating 22, containing a catalyst capable of converting the species $S_2$ to $S'_2$. The $n^{th}$ specific working electrode is covered by the membrane coating 2n which comprises a catalyst capable of converting the species $S_n$ into the species $S'_n$. Preferably, the catalysts comprised in the membrane coatings 21, 22, ... , 2n are only capable of converting one specific species and do not catalyse the conversion of any other species present in the fluid.

In summary, the arrangement shown in Figure 9 comprises n+1 working electrodes and allows a simultaneous measurement of concentrations of species $S_1$, $S_2$, ... , $S_n$ in the fluid 5.

**Advantages provided by the present invention**

The present invention allows an efficient detection and *in situ* monitoring of a broad range of ROS and RNS, in particular of RNS such as $NO_x$, or HNO. This has been demonstrated by monitoring of HNO release from AS. Similar sensors can also be used for monitoring HNO release from living cells. Presently, bio-sample preparation before the measurement is a very common practice in measuring reactive nitrogen and oxygen species. However, using the sensor of the present invention allows avoiding the sample preparation step and enables a direct integration of the sensor into the tissue environment. Unlike other electrochemical RNS measurement techniques, the sensor of the present invention allows a selective detection of the species of interest (analyte compound) in the presence of interfering compounds due to the differential signal measurement ($\Delta I$). Other significant advantages of the sensor of the present invention are its quick response time and ability to conduct aerobic, anaerobic and gas phase measurements in a broad range of applications (e.g. *in vitro, in vivo*).

In particular, the sensors of the present invention allow detection and quantification of the analyte compound such as HNO in the presence of a detection compound such as NO. The concentration of the analyte compound in the fluid may be as low as 1 mM, preferably as low as 100 $\mu$M, more preferred as low as 10 $\mu$M, even more preferred as low as 1 $\mu$M and particularly preferred as low as 10 nM. Moreover, the sensors of the present invention allow detection and quantification of the analyte compound in concentrations up to 10 M.

Another advantage of the sensor of the present invention is that it can be built in a highly compact manner. The sensor of the present invention typically has a width of from 2 mm to 10 mm, for instance 5.4 mm, a length of from 10 mm to 50 mm, more preferred of from 20 mm to 30 mm, for instance 24 mm. The sensor of the present invention preferably has a thickness of from 500 $\mu$m to 700 $\mu$m, for instance about 500 $\mu$m.

The sensor of the present invention may contain multiple working, counter and reference electrodes. For example, the sensor having a size of ca. 5.4 x 24 mm, can have six (3 x 2) working electrodes, two reference electrodes and one counter electrode.

The diameter of the wafer for the manufacture of the sensor typically ranges between 50 mm and 200 mm and can be for example about 100 mm. The wafer may have a thickness of 2 to 7 mm e.g. about 3 or 5 mm. Alternatively, the wafer may have a thickness of 200 $\mu$m to 2 mm, preferably from 300 $\mu$m to 800 $\mu$m and especially preferred about 500 $\mu$m. The wafer is cut into several sensing chips (sensors).

**Examples**

**Example 1: Preparation of the HNO sensor used in the present invention**

The sensor utilised in the present example was fabricated on a Pyrex® wafer substrate (100 mm diameter and 500 $\mu$m thickness), where silicon nitride and silicon oxide layers act as the isolation layer. Thin-film platinum was chosen as material for both working electrodes (200 $\mu$m diameter). One of the Pt working electrodes was coated with the inert membrane coating (polyHEMA: Pt/Blank) and the other working electrode was coated with SOD enzyme mixed with polyHEMA (Pt/SOD, 0.16 wt.-%). This embodiment did not contain any oxygen impermeable membrane coating (e.g. xerogels) as this experiment was meant to detect the presence of HNO in aerobic environment. Built-in platinum (Pt) and silver/silver chloride (Ag/AgCl) electrodes acted as the counter and the reference electrodes, respectively.
In the following, the preparation procedure of the sensor will be described in detail.

**Substrate**

A Pyrex wafer (Borofloat®) having 100 mm diameter, 500 $\mu$m thickness was used. The deposition of silicon nitride ($Si_xN_y$) on the wafer to a 500 nm thick layer was carried out by plasma enhanced chemical vapour deposition (PECVD).

**Electrode material deposition**

- Spin coating and development of negative photoresist AZ 5214E on $Si_xN_y$.
- Physical vapour deposition of Ti/Pt/Ti 50/150/20 nm thickness.
- Lift-off the photoresist with acetone and isopropanol to make the metal structures.

**Isolation layer deposition**

Above structured metal $Si_xN_y$ was deposited by PECVD, at mixed frequency, T=300°C. Subsequently, silicon oxide (SiO) was deposited as PECVD, low frequency, T=300°C. Photolithography and reactive ion etching of $Si_xN_y$ + SiO + Ti; etch depth 800 + 200 + 20 nm.
Pt electrodes were used as the working electrodes (the first working electrode and the second working electrode, both having diameter of 200 $\mu$m) and the counter electrode (400 $\mu$m diameter). Similarly, a Pt electrode (200 $\mu$m diameter) was also used for a subsequent deposition of the Ag/AgCl layers to serve as the reference electrode.

**Wafer level fabrication of the built-in reference electrode**

**Silver deposition**

Electrolyte: 30 ml Arguna S, 143 g potassium cyanide (98-99 wt.-%), 74 g potassium silver cyanide (54 wt.-%), 5 g potassium hydroxide.
Wafer as cathode: Pt
Auxiliary electrode: silver wire
Current density: 16 mA/cm$^2$ for 10 minutes (ultrasonication).

**Silver chloride deposition**

Electrolyte: 0.1 ml KCl bath
Wafer as anode: Pt/Ag,
Auxiliary electrode: Pt wire
Current density: 1.6 mA/cm$^2$ for 10 minutes (ultrasonication).

**Dicing wafer into sensing chips**

The wafer was cut using a diamond dicer from the back end of the wafer.

**Composition of hydrogel used for the membrane coatings and dispension on the working electrodes**

The components used for the preparation of hydrogel as well their contents are listed in the Table 1 below.

Table 1

| Components of the Hydrogel | Weight ratio (wt.-%) | Purpose | Purchased from |
|---|---|---|---|
| 2-hydroxyethylmethacrylate (HEMA) | 20 | reactive monomer | VWR International GmbH |
| Poly(2-hydroxyethyl methacrylate) polyHEMA | 28 | polymeric binder | Sigma Aldrich |
| ethylene glycol | 29.6 | plasticizer | Fluka |
| triethylene glycol dimethacrylate (TEGDMA) | 2.2 | crosslinker | Polysciences |
| Irgacure® 651 (2,2-dimethoxy-1,2-di(phenyl)ethanone) | 0.8 | photoinitiator | Sigma Aldrich |
| phosphate buffer saline (PBS) | 19.4 | | |

Dispension of Hydrogel

Device: pressure dispenser (SPETEC GmbH)
Needle inner diameter: 0.004 inch (ca. 102 $\mu$m)

Hydrogel on the counter and the reference electrodes
dispensing parameters: dispensing pressure - 1.1 bar, duration - 1 s

Hydrogel on the first working electrode
dispensing parameters: 0.35 bar, duration 500 ms

Hydrogel on the second working electrode
0.16 wt% of SOD mixed with hydrogel
dispensing parameters: 0.35 bar, duration 500 ms

After dispensing the hydrogel, it was immediately brought for UV curing in oxygen-free environment (2 minutes ($N_2$ Supply) + 3 minutes (UV)). The dispensed and cured hydrogels were dipped into a chamber containing PBS (~12 hrs).

**Example 2: Measurement of HNO formed by decomposition of AS in fluid**

**Materials required for detecting and measuring HNO**

A 2 ml reaction vessel (BRAND GmbH & Co KG) was filled with 1.5 ml of phosphate buffer saline (PBS, pH 7.4). The solution was stirred by a magnetic stirrer 2.1 V DC. The HNO sensor prepared in Example 1 was dipped into the measurement solution. Long-term amperometric measurement was executed and waited until a constant baseline current was achieved. Fresh sample of Angeli's salt (AS, sodium $\alpha$-oxyhyponitrite) obtained from Cayman Chemical (Cayman Chemical Company, Michigan, USA) was dissolved in a separate PBS solution (1 ml reaction vessel). A precise volume of AS dissolved PBS was added to the measurement chamber to form the total concentration as 147 $\mu$M of AS.

For measuring HNO concentration series, a precise volume of AS dissolved PBS was added soon after the preparation to the measurement chamber at the interval of 5 minutes and the corresponding current due to oxidation was measured.

**Method used for measuring HNO**

All experiments were conducted using the HNO sensor. Built-in Pt and Ag/AgCl electrode acted as the counter and the reference electrodes, respectively. Amperometric experiments were conducted using Metrohm Autolab Potentiostat/Galvanostat. All experiments were started only after observing the change in open circuit potential was as low as 100 $\mu$V/s. The working electrodes (the first working electrode and the second working electrode) were pretreated by applying a potential of 940 mV vs. Ag/AgCl. Subsequently, the measurement was started by applying potential of 880 mV on the working electrodes. Choice of 880 mV was due to the direct oxidation of NO and nitrite ions against Ag/AgCl electrode at the pH 7.4. The acquisition rate for the measurement was 1 s.

The whole experiment was conducted under a room temperature of 24°C, atmospheric pressure of 101 kPa and at ambient oxygen environment. Angeli's salt (AS) was used as a donor of HNO. AS decomposes into HNO and $NO_2^-$ under physiological pH as shown in the equation 4.

$$N_2O_3^{2-} + H^+ \rightarrow HNO + NO_2^- \qquad (4)$$

In this experiment, a defined amount of AS dissolved in PBS was directly added to the phosphate buffered saline (PBS, pH 7.4), which decomposed to form HNO. The concentration of AS was 147 $\mu$M. A potential of 880 mV vs. built-in Ag/AgCl was applied to both working electrodes and the corresponding currents were measured using Metrohm Autolab Potentiostat/Galvanostat.

The grey and black curves in Figure 5 denote the signal of the first working electrode coated with polyHEMA (Pt/Blank) and the signal of the second working electrode, which was coated with SOD enzyme mixed with polyHEMA (Pt/SOD), respectively. Less than 500 pA (0.002 mA/cm$^2$) current was observed before the addition of AS ($I_{bl}$). Soon after the addition of AS, a quick signal increase was observed at both working electrodes.

The possible current contribution ($I_x$) is from oxidation of Angeli's salt and its products (HNO and $NO_2^-$) on the surface of the working electrodes. Moreover, $N_2O$, which is formed by dimerisation of HNO is also expected to influence the current (see equation 1). The total current at Pt/Blank electrode after the addition of Angeli's salt can be given as

$$I_{Pt/Blank} = I_{bl} + I_X \qquad (5)$$

The signal of the Pt/SOD electrode exhibited a higher increase than the signal of the Pt/Blank electrode. The significant signal rise particularly after the addition of AS was due to the signal dependent on NO. As explained earlier (equation 3), this signal is expected to result from the enzymatic conversion of HNO to NO by the SOD enzyme. The general oxidation current due to NO is given as follows

$$NO + 2H_2O \rightarrow NO_3^- + 3e^- + 4H^+ \qquad (6)$$

So, the total current at the Pt/SOD electrode can be given as

$$I_{PT/SOD} = I_{BL} + I_X + I_{NO} \qquad (7)$$

Thus, the signal difference between two working electrodes is dependent on concentration of HNO as shown in the equation below.

$$\Delta I = I_{Pt/SOD} - I_{Pt}/Blank = I_{NO} \rightarrow [HNO] \qquad (8)$$

The difference in signal was gradually reduced over time due to the decay of the produced NO. Also the signal on both working electrodes decreased due to the decay of $N_2O$ formed after dimerisation of HNO. After about seven hours both signals attain the stable current ($I_{bl}$) without significant difference. The result also shows the long-term stability of both working electrodes while monitoring the decomposition of AS.

**Example 3: Measurement of AS dependent HNO concentration in fluid**

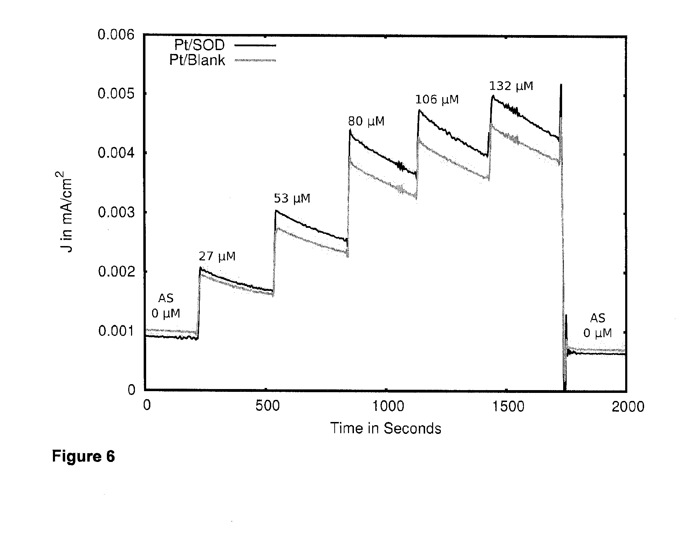

In order to further evaluate the performance of the sensing principle of HNO, the measurements were carried out at different AS concentrations (Figure 6). The raw data (shown as bright grey background) were subjected to fast Fourier Transformation (FFT) filtering with n=5. The experiments were carried out as described in Example 2 above.
A potential of 880 mV vs. built-in Ag/AgCl was applied to both working electrodes until a baseline current was achieved (~0.001 mA/cm$^2$). Subsequently, the initial concentration of HNO was achieved by addition of AS (27 $\mu$M AS). The signal of the electrode coated with SOD enzyme (Pt/SOD) was higher than the signal of the blank electrode (Pt/blank).
This signal difference increased with larger concentrations of AS. After the addition of 132 $\mu$M AS (~1500 s), the observed signal difference was about 0.5 $\mu$A/cm$^2$. Angeli's salt life-time in PBS, its concentration range and signal difference at 132 $\mu$M are in good agreement with the result presented in Figure 5. The measurement of the HNO concentration could be carried out in a reproducible and selective manner.

**Example 4: Evaluation of interference elimination (NO$_2$$^-$)**

**Materials required for measuring nitrite ions**

A 2 ml reaction vessel (BRAND GmbH & Co KG) was filled with 1.5 ml of phosphate buffer saline (PBS, pH 7.4). The solution was stirred by a magnetic stirrer 2.1 V DC. HNO sensor prepared in Example 1 was dipped into the measurement solution. Long-term amperometric measurement was executed and waited until a constant baseline current was achieved. Sodium nitrite salt was weighed and was dissolved in a separate PBS solution. A precise volume of sodium nitrite dissolved PBS was added to the measurement chamber at the interval of 5 minutes and the corresponding current due to oxidation was measured.
Nitrite ions are considered to be the stable and final product of decomposition of many dissolved RNS including HNO. In the present investigation, nitrite ions are also formed as co-product during the decomposition of Angeli's salt. Moreover, similar to nitric oxide, nitrite ions are capable of undergoing anodic oxidation to nitrate ions at the same redox potential (~810 mV vs. Ag/AgCl electrode)

$$NO_2^- + H_2O - 2H^+ + NO_3^- + 2e^- \qquad (9)$$

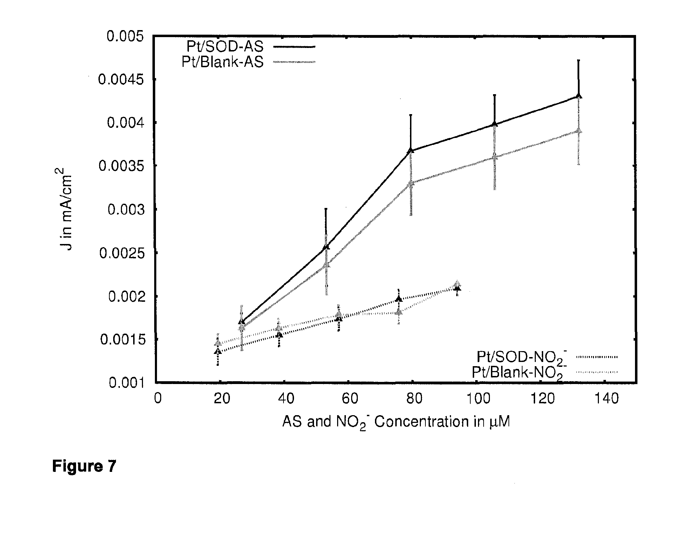

The inherent presence and inability to distinguish the redox potential from NO made nitrite ions a chief interfering molecule for measuring HNO.
Thus, in order to evaluate the selectivity of the differential biosensor of the present invention, the concentration plots of AS (upper pair in Figure 7) were compared to the concentration plot of nitrite ions (addition of NaNO$_2$ in PBS, lower pair in Figure 7). The result presented in Figure 7 is the outcome of the evaluation of a set of working electrodes (Pt/SOD in black & Pt/Blank in gray) performance at two different experimental conditions. They are the experiment performed by the sequential addition of AS dissolved in PBS (continuous lines in Figure 7, extracted from Figure 6) and the experiment performed by the sequential addition of NaNO$_2$ dissolved in PBS (discontinuous lines in Figure 7). The average current was plotted 300 s after the addition of salts (n=10), where "n" is the number of data points taken at each 300 seconds.
The current density from the pair of sensing electrodes deviates from each other upon addition of Angeli's salt (continuous lines). As expected, SOD coated Pt electrode shows more current than Pt/Blank electrode when more AS is added (black vs. gray). But in the other experiment (discontinuous lines), the current density is constant upon addition of concentration of nitrite ions in the same range. From this result it becomes evident that nitrite ions did not influence the current difference of Angeli's salt.
To date, electrochemical nitric oxide sensors are still vulnerable to nitrite ions (lack of selectivity). The presented results illustrate that the sensor of the present invention is capable to selectively determine the HNO concentration the presence of nitrite ions. From this result it is also evident that the influence of any other biologically relevant species does not have influence on the HNO detection and HNO measurements as long as these species does not affect the catalyst, e.g. the SOD enzyme. Thus, the results demonstrate the selectivity of the sensor of the present invention for a broad range of analyte compounds.
A further look on the results shows a non-linear behaviour of the set of working electrode upon addition of PBS dissolved Angeli's salt (continuous lines). One significant reason is illustrated by equation 4. AS exhibits quick reactivity in excessive hydrogen ion concentrations (PBS, pH 7.4 as stock solution for measurement), which in-turn decomposes the significant amount of AS dissolved in PBS over time. Thus, the time dependent linearity was observed even at higher concentration of AS. However, this characteristic behaviour was not observed when PBS dissolved in nitrite was added (discontinuous lines).

**Claims**

1. A sensor for a selective electrochemical detection of an analyte compound in a fluid, the sensor comprising:

    a) a first working electrode,
    b) a second working electrode covered by a second membrane coating, whereby the second working electrode is in proximity to a catalyst and the second membrane coating is permeable for the analyte compound,

    the catalyst is capable of catalysing the conversion of the analyte compound into a detection compound,
    the detection compound can be detected by the first working electrode and by the second working electrode and
    the analyte compound is selected from the group consisting of reactive oxygen species and reactive nitrogen species.

2. Sensor according to claim 1, whereby the first working electrode is covered by a first membrane coating.

3. Sensor according to claim 2, whereby the first membrane coating is permeable for the analyte compound and for the detection compound.

4. Sensor according to any of claims 1 to 3, whereby the analyte compound is nitroxyl and the detection compound is nitric oxide.

5. Sensor according to any of claims 1 to 4, whereby the first working electrode is covered by a first membrane coating, the analyte compound is nitroxyl and the detection compound is nitric oxide.

6. Sensor according to any of claims 1 to 5, whereby the first working electrode and the second working electrode comprise a material selected from the group consisting of carbon, indium tin oxide, iridium oxide, nickel, platinum, silver and gold.

7. Sensor according to any of claims 1 to 6, whereby the first membrane coating and the second membrane coating comprise a membrane impermeable for molecular oxygen.

8. Sensor according to any of claims 1 to 7, whereby the catalyst is an enzyme.

9. Sensor according to any of claims 1 to 8, whereby the catalyst is a Cu-Zn superoxide dismutase.

10. Sensor according to any of claims 1 to 9, whereby the first membrane coating and the second membrane coating further comprise an additional catalyst.

11. Sensor according to any of claims 1 to 10, whereby the sensor further comprises a counter electrode.

12. Sensor according to any of claims 1 to 11, whereby the sensor further comprises a reference electrode.

13. Use of a sensor according to any of claims 1 to 12 for a selective electrochemical detection of the analyte compound in the fluid.

14. Use according to claim 13, whereby detection of electrical signals from the first working electrode and from the second working electrode is carried out by an analytical method selected from the group consisting of amperometry, potentiometry, square wave voltammetry, differential pulse amperometry and differential pulse voltammetry.

15. Use according to claim 13 or 14, wherein the fluid comprising the analyte is a biological fluid.

**Figure 1**

**Figure 2**

**Figure 3**

**Figure 4**

**Figure 5**

**Figure 6**

**Figure 7**

Figure 8

Figure 9

**EUROPEAN SEARCH REPORT**

Application Number

EP 12 17 3383

Europäisches
Patentamt
European
Patent Office
Office européen
des brevets

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WANSIK CHA ET AL: "Patterned Electrode-Based Amperometric Gas Sensor for Direct Nitric Oxide Detection within Microfluidic Devices", ANALYTICAL CHEMISTRY, vol. 82, no. 8, 15 April 2010 (2010-04-15), pages 3300-3305, XP55041576, ISSN: 0003-2700, DOI: 10.1021/ac100085w * figure 1 * | 1 | INV. G01N33/543 |
| X,D | ARAVINDALOCHANAN K ET AL: "Simulation and design of a nitric oxide sensor array for cell cultures", SENSORS, 2009 IEEE, IEEE, PISCATAWAY, NJ, USA, 25 October 2009 (2009-10-25), pages 325-328, XP031618557, ISBN: 978-1-4244-4548-6 * the whole document * | 1-15 | |
| X,D | ARAVINDALOCHANAN K ET AL: "Optimising a Nitric oxide sensing technique for hypoxic tumor cell cultures", SOLID-STATE SENSORS, ACTUATORS AND MICROSYSTEMS CONFERENCE, 2009. TRANSDUCERS 2009. INTERNATIONAL, IEEE, PISCATAWAY, NJ, USA, 21 June 2009 (2009-06-21), pages 1003-1006, XP031545868, ISBN: 978-1-4244-4190-7 * the whole document * -/-- | 1-15 | TECHNICAL FIELDS SEARCHED (IPC) G01N |

The present search report has been drawn up for all claims

| Place of search | Date of completion of the search | Examiner |
|---|---|---|
| Munich | 22 October 2012 | Hinchliffe, Philippe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EUROPEAN SEARCH REPORT

Application Number

EP 12 17 3383

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SEBASTIÁN A. SUÁREZ ET AL: "A Surface Effect Allows HNO/NO Discrimination by a Cobalt Porphyrin Bound to Gold", INORGANIC CHEMISTRY, vol. 49, no. 15, 2 August 2010 (2010-08-02), pages 6955-6966, XP55041559, ISSN: 0020-1669, DOI: 10.1021/ic1007022 * page 6966, column 1 - column 2 * | 1-15 | |
| A | YOUNGMI LEE ET AL: "Simultaneous Electrochemical Detection of Nitric Oxide and Carbon Monoxide Generated from Mouse Kidney Organ Tissue", ANALYTICAL CHEMISTRY, vol. 79, no. 20, 15 October 2007 (2007-10-15), pages 7669-7675, XP55041574, see results section; * abstract * | 1-15 | |
| A | A. ZELLER ET AL: "Mechanisms Underlying Activation of Soluble Guanylate Cyclase by the Nitroxyl Donor Angeli's Salt", MOLECULAR PHARMACOLOGY, vol. 76, no. 5, 31 August 2009 (2009-08-31), pages 1115-1122, XP55041746, ISSN: 0026-895X, DOI: 10.1124/mol.109.059915 * see discussion * | 1-15 | TECHNICAL FIELDS SEARCHED (IPC) |

-/--

The present search report has been drawn up for all claims

| Place of search | Date of completion of the search | Examiner |
|---|---|---|
| Munich | 22 October 2012 | Hinchliffe, Philippe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

Europäisches
Patentamt

European
Patent Office

Office européen
des brevets

**EUROPEAN SEARCH REPORT**

Application Number

EP 12 17 3383

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SALMON DEBRA J ET AL: "Comparison of HNO detection methods for quantitative results", AMERICAN CHEMICAL SOCIETY. ABSTRACTS OF PAPER. AT THE NATIONAL MEETING, AMERICAN CHEMICAL SOCIETY, US, vol. 237, 1 January 2009 (2009-01-01), page INOR459, XP009163883, ISSN: 0065-7727 * abstract * | 1-15 | |

-----

| | | | TECHNICAL FIELDS SEARCHED (IPC) |
|---|---|---|---|
| | | | |

The present search report has been drawn up for all claims

| Place of search | Date of completion of the search | Examiner |
|---|---|---|
| Munich | 22 October 2012 | Hinchliffe, Philippe |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5603820 A

**Non-patent literature cited in the description**

- **V. SHAFIROVICH et al.** *Proc. Nat. Acad. Sci. USA,* 2002, vol. 99 (11), 7340-7325
- **P. A. S. SMITH et al.** *J. Am. Chem. Soc.,* 1960, vol. 82 (21), 5731-5740
- **J. M. FUKUTO et al.** *Biochem. Pharmacol.,* 1992, vol. 43 (3), 607-613
- **B. SHEN et al.** *Biochemistry,* 2005, vol. 44 (42), 14030-14044
- **M. A. MARTI et al.** *J. Am. Chem. Soc.,* 2005, vol. 127 (12), 4680-4684
- **K. P. DOBMEIER et al.** *Anal. Chem.,* 2008, vol. 80 (4), 1247-1254
- **J. M. FUKUTO et al.** *Chem. Bio. Chem.,* 2005, vol. 6 (4), 612-619
- **J. C. IRVINE et al.** *Trends Pharmacol. Sciences,* 2008, vol. 29 (12), 601-608
- **N. PAOLOCCI et al.** *Pharmacology & Therapeutics,* 2007, vol. 113 (2), 442-458
- **E. NOVO et al.** *Fibrinogenesis and Tissue Repair,* 2008, vol. 1, 5
- Polymers, electrically conductive. **H. NAARMANN.** Ullmann's Encyclopedia of Industrial Chemistry. 1992, vol. A21, 429
- **W. J. KORROS et al.** *Pure & Appl. Chem.,* 1996, vol. 68 (7), 1479-1489
- **ZHANG et al.** *Electrochem. Commun,* 2002, vol. 4, 11-16
- **K. ARAVINDALOCHANAN et al.** *Solid-State Sensors, Actuators and Microsystems Conference,* 2007, 1907-1910
- **P. EBBESEN.** *Journal of Enzyme Inhibition and Medicinal Chemistry,* 2009, vol. 24 (1), 1-39